# EUROPÄISCHE PATENTSCHRIFT

(11) **EP 1 200 452 B1**
(45) Veröffentlichungstag und Bekanntmachung des Hinweises auf die Patenterteilung: **26.03.2003**
(21) Anmeldenummer: 00956258.8
(22) Anmeldetag: 22.07.2000
(51) Int. Cl.: C07F 9/60, C07F 9/6571, B01J 31/24, C07B 53/00, C07C 45/50

(54) **NEUE CHIRALE PHOSPHORLIGANDEN UND IHRE VERWENDUNG IN DER HERSTELLUNG OPTISCH AKTIVER PRODUKTE**
NOVEL CHIRAL PHOSPHORUS LIGANDS AND THE USE THEREOF IN THE PRODUCTION OF OPTICALLY ACTIVE PRODUCTS
NOUVEAUX LIGANDS CHIRAUX DE PHOSPHORE ET LEUR UTILISATION DANS LA PRODUCTION DE PRODUITS OPTIQUEMENT ACTIFS

(30) Priorität: 03.08.1999 DE 19936473
(43) Veröffentlichungstag der Anmeldung: 02.05.2002
(73) Patentinhaber: Studiengesellschaft Kohle mbH, 45470 Mülheim (DE)
(72) Erfinder: LEITNER, Walter, D-45470 Mülheim/Ruhr (DE); FRANCIO, Giancarlo; Universität von Messina, ica, Chimica Fisica; Salita Sperone, 31, (IT); FARAONE, Felice; Universität von Messina, ica, Chimica Fisica; Salita Sperone, 31, (IT); ARENA, Carmela, G.; Universität von Messina, ica, Chimica Fisica; Salita Sperone, 31, (IT)
(74) Vertreter: von Kreisler, Alek, Dipl.-Chem.
(86) Internationale Anmeldenummer: EP0007053
(87) Internationale Veröffentlichungsnummer: WO01009147

(56) Entgegenhaltungen:
- WO-A-93/03839
- FRANCIµ G. ET AL.: "Chiral phosphamidite ligands based on 8-chloroquinoline and their Rhodium(III), Palladium(II) and Platinum(II) complexes " EUROPEAN JOURNAL OF INORGANIC CHEMISTRY., Nr. 8, 20. Juli 1999 (1999-07-20), Seiten 1219-1227, XP002151551 WILEY-VCH VERLAG, WEINHEIM., DE ISSN: 1434-1948 in der Anmeldung erwähnt
- DOUCET H. ET AL.: "The scope of catalytic asymmetric hydroboration/oxidation with rhodium complexes of 1,1'-(2-diarylphos- phino-1-naphtyl)isoquinolines" CHEMISTRY - A EUROPEAN JOURNAL., Bd. 5, Nr. 4, April 1999 (1999-04), Seiten 1320-1330, XP002151552 VCH PUBLISHERS., US ISSN: 0947-6539
- NOZAKI K.: "Highly enantioselective hydroformylation of olefins catalyzed by Rhodium(I) complexes of new chiral phosphine-phosphite ligands" JOURNAL OF THE AMERICAN CHEMICAL SOCIETY., Bd. 119, Nr. 19, 14. Mai 1997 (1997-05-14), Seiten 4413-4423, XP002151553 AMERICAN CHEMICAL SOCIETY, WASHINGTON, DC., US ISSN: 0002-7863
- BRUNEL J M ET AL: "Enantioselective Copper Catalyzed Diels-Alder Reaction Using Chiral Quinoline-Phosphine Ligand" TETRAHEDRON LETTERS,NL,ELSEVIER SCIENCE PUBLISHERS, AMSTERDAM, Bd. 39, Nr. 52, 24. Dezember 1998 (1998-12-24), Seiten 9663-9666, XP004144285 ISSN: 0040-4039
- FRANCIµ, G. ET AL: "Asymmetric catalysis with chiral phosphane/ phosphoramidite ligands derived from quinoline (QUINAPHOS)" ANGEW. CHEM., INT. ED. 39(8), 1428-1430 , 17. April 2000 (2000-04-17), XP002151554

## Beschreibung

Die vorliegende Erfindung betrifft neue chirale Phosphorverbindungen, die sich in einfacher Weise aus Chinolinderivaten darstellen lassen sowie deren Verwendung als Katalysatoren oder Katalysatorbestandteile in Verfahren zur Herstellung optisch aktiver Produkte.

Chirale Phosphorverbindungen sind als Katalysatoren oder Katalysatorbestandteile ("Liganden") für die enantioselektive chemische Synthese optisch aktiver Produkte von großem Interesse (*Handbook of Enantioselective Catalysis with Transition Metal Compounds, Vol. II*, VCH, Weinheim, **1993**). Optisch aktive Produkte haben große wirtschaftliche Bedeutung als Aromastoffe, Kosmetika, Pflanzenschutzmittel, Lebensmittelzusatzstoffe, Pharmazeutika oder in der Herstellung von High-Tech Werkstoffen wie z.B. Spezialkunststoffen *(Comprehensive Asymmetric Catalysis,* Springer, Berlin, **1999**). Trotz der Vielfalt bekannter chiraler Phosphorverbindungen haben bislang nur wenige Vertreter Verwendung in industriellen Prozessen zur Herstellung optisch aktiver Produkte gefunden, da viele Liganden für technische Anwendungen gravierende Nachteile aufweisen. Viele Liganden weisen zwar hohe Enantioselektivitäten auf, bilden die gewünschten chiralen Produkte aber mit zu geringen Aktivitäten oder unzureichenden Chemo- oder Regioselektivitäten. Chirale Phosphorverbindungen, die als effiziente Liganden wirken, sind femer vielfach nur über aufwendige Synthesen unter Verwendung teurer Ausgangsmaterialien zugänglich. Die chirale Information, die zur selektiven Bildung der optisch aktiven Produkte führt, beruht bei den meisten effizienten Liganden auf der Verwendung chiraler Bausteine, die entweder von natürlich vorkommenden Verbindungen abgeleitet sind, oder anderweitig in enantiomerenreiner Form kommerziell verfügbar sind. Eine strukturelle Variation am chiralen Zentrum zur Optimierung der Phosphorverbindung ist dann in einfacher Form nicht realisierbar und häufig steht nur eine der beiden möglichen Konfigurationen zur Verfügung. Es besteht daher ein großer Bedarf an neuen chiralen Phosphorverbindungen, die sich in einfacher und flexibler Weise aus leicht zugänglichen und billigen Ausgangsverbindungen synthetisieren lassen und die als Katalysatoren oder Katalysatorbestandteile für die Herstellung chiraler Produkte in verschiedenen Reaktionstypen effektiv eingesetzt werden können.

Die vorliegende Erfindung betrifft eine neue Klasse von chiralen Phosphorverbindungen der allgemeinen Formel **I**, 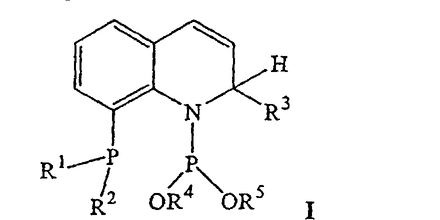 wobei R¹, R², R³, R⁴, R⁵ chirale oder achirale organische Reste sind, die sich von substituierten oder unsubstituierten geradkettigen, verzweigten, oder cyclischen aliphatischen oder aromatischen Gruppen ableiten und die im Falle der Paare R¹/R² und R⁴/R⁵ miteinander verbunden sein können. Diese Verbindungen lassen sich in einfacher Weise und in wenigen Schritten ausgehend von Derivaten des Chinolins als billigen Ausgangsmaterialen darstellen. Die für die selektive Bildung der gewünschten optisch aktiven Produkte ausschlaggebende chirale Information in 2-Position des Chinolingerüsts wird während der Synthese erzeugt und kann leicht über die Wahl von R³ variiert werden. Die beiden Isomeren mit den unterschiedlich Konfigurationen in 2-Position lassen sich in effektiver Weise voneinander trennen. Die Verbindungen der Formel **I** können als effiziente Katalysatoren oder Katalysatorbestandteile in der Herstellung optisch aktiver Produkte eingesetzt werden, wobei insbesondere in der enantioselektiven Hydroformylierung und Hydrierung hohe Aktivitäten und Selektivitäten erzielt werden.

### Synthese der Phosphorverbindungen I:

Zur Synthese der Phosphorverbindungen **I** (Schema 1) geht man zweckmäßig von 8-Phosphinochinolinen **II** aus. Verbindungen **II** sind für verschiedene Reste R¹ und R² bereits bekannt und lassen sich auf verschiedenen Wegen einfach im Multigramm-Maßstab herstellen (typische Beispiele: *Inorg. Chem.* 1982, *21,* 1007; *J. Organomet. Chem.* **1997,** *535,* 183). Mithilfe dieser Synthesen und geeigneten einfachen Modifikationen können Verbindungen vom Typ **II** hergestellt werden, bei denen R¹ oder R² identische oder voneinander verschiedene, chirale oder achirale organische Reste sind, die sich von substituierten oder unsubstituierten geradkettigen, verzweigten, oder cyclischen aliphatischen oder aromatischen Gruppen ableiten, und die miteinander verbunden sein können. Die Reste R¹ und R² können unabhängig voneinander gewählt werden aus den Gruppen Methyl, Ethyl, n-Propyl, n-Butyl, Hexyl, F(CF₂)ₘ(CH₂)ₙ-(m=1-10, n=0-4), Cyclohexyl, Menthyl, Allyl, Benzyl, CH₃O(CH₂)₂OCH₂-, Phenyl, Tolyl, Anisyl, Trifluormethylphenyl, F(CF₂)ₘ(CH₂)ₙC₆H₄- (m = 1-10, n = 0-4), Bis-(trifluormethyl)-phenyl, Chlorphenyl, Pentafluorphenyl, Hydroxyphenyl, Carboxyphenyl, NaO₃SC₆H₄-, Naphthhyl, Fluorenyl, Pyridyl oder Furyl ohne daß durch die genannten Gruppen eine Beschränkung der Anwendungsbreite impliziert werden soll. Wenn die beiden Gruppen miteinander verbunden werden, können substituierte oder unsubstituierte, chirale oder achirale Brücken entstehen, die sich z.B. von den Gerüsten -(CH₂)ₙ- (n=2-4), -CH(CH₃)CH(CH₃)-, -CH(CH₃)CH₂CH(CH₃)-, 1,1'-Biphen-2,2'-diyl oder 1,1'-Binaphth-2,2'-diyl ableiten, wobei auch hier durch die Auflistung keine Einschränkung impliziert wird.
Die Umsetzung von **II** mit Nukleophilen Reagentien R³M liefert die Verbindungen **III,** wobei R³ der gleichen Definition wie R¹ oder R² entspricht. Die Addition in 2-Position des Chinolins ist mit Grignard-Verbindungen (M = MgHal, Hal = Halogen) und vielen anderen Organometallverbindungen (z:B. M = Li, ZnR, SnR₃; R = Alkyl oder Arylrest) möglich, so daß eine große Variationsbreite möglicher Derivate resultiert. Bei der Addition in 2-Position des Chinolins wird ein chirales Zentrum erzeugt, wobei die Stereochemie an diesem Zentrum in

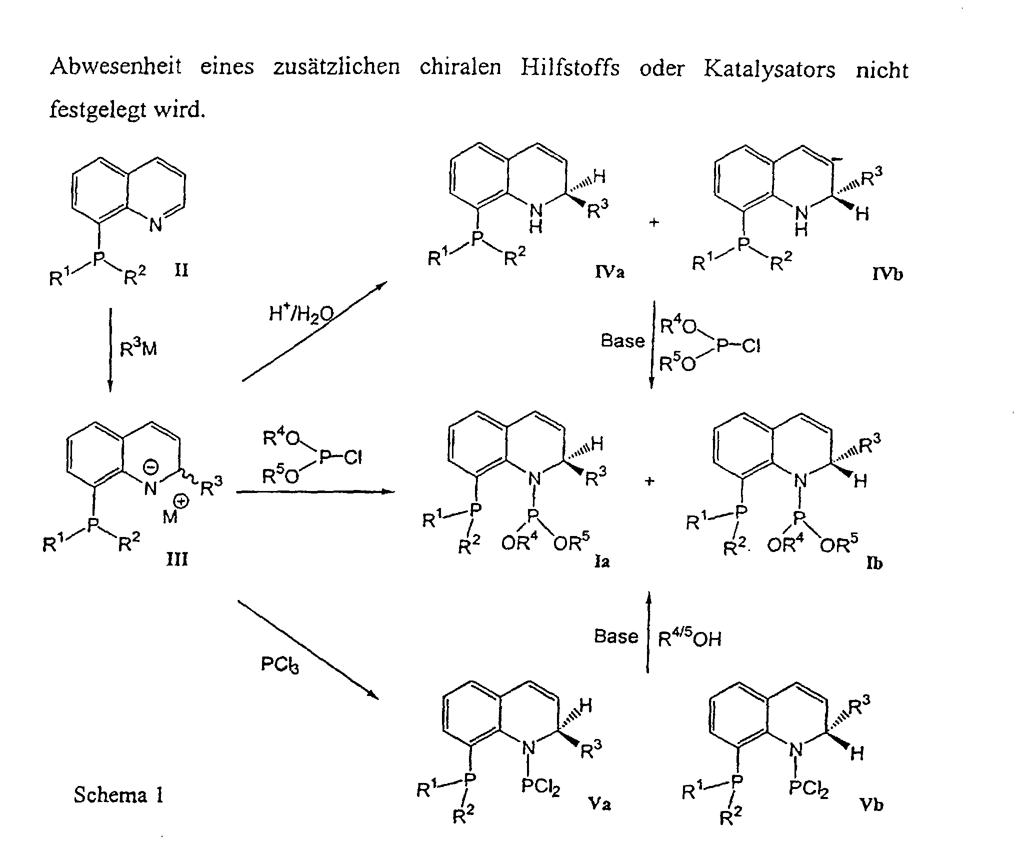

Durch Hydrolyse lassen sich die Verbindungen **III** in die 1,2-Dihydrochinolinderivate **IV** überführen. Reaktion mit Chlorophosphiniten (R⁴O)(R⁵O)PCl in Gegenwart von Basen wie z.B. Triethylamin oder Pyridin ergibt die gewünschten Phosphorverbindungen der Formel **I.** Ein alternativer Zugang besteht in der Reaktion von **III** mit PCl₃, wobei die Dichlorophosphinderivate **V** entstehen. Umsetzung mit Alkoholen oder Diolen in Gegenwart von Base liefert wiederum **I.** Die Verbindungen **III** können auch direkt mit Chlorophosphiniten (R⁴O)(R⁵O)PCl ohne weiteren Zusatz von Basen zu **I** umgesetzt werden.

Die Reste R⁴ und R⁵ können gleich oder verschieden, achiral oder chiral, und miteinander verbunden sein. Ansonsten weisen die Reste die gleiche Definition wie die Reste R¹ und R² auf. Beispiele für Alkohole und Diole, die zur Herstellung entsprechenden Verbindungen (R⁴O)(R⁵O)PCl verwendet oder direkt mit **V** umgesetzt werden können sind Methanol, Ethanol, *iso*-Propanol, Benzylalkohol, Cyclohexanol, Allylalkohol, Phenol, Methylphenol, Chlorphenol, Naphthol, Furfurol, Ethylenglycol, 1,3-Propandiol, 1,3-Pentandiol, Cyclohexandiol, Glycerin, Monosaccharide, Oligosaccharide, Catechol, 2,2'-Dihydroxy-1,1'-Biphenyl, 3,3',5,5'-Tetra*-tert*.-Butyl-2,2'-Dihydroxy-1,1'-Biphenyl, 3,3'-Di-*tert*.-Butyl-2,2'-Dihydroxy-5,5'-Dimethoxy- 1,1'-Biphenyl, 5,5'-Dichloro-4,4',6,6'-Tetramethyl-2,2'-Dihydroxy-1,1'-Biphenyl, oder 2,2'-Dihydroxy-1,1'-Binaphthyl, wobei durch die Auflistung keine Einschränkung der Anwendungsbreite impliziert werden soll.
Bei Verwendung optisch aktiver (R⁴O)(R⁵O)P-Gruppen erhält man die Verbindungen **I** als Diastereomere, die sich durch Kristallisation, Chromatographie oder andere geeignete Trennmethoden separieren lassen. Alternativ kann die Trennung der beiden Stereoisomeren auf der Stufe der 1,2-Dihydrochinolinderivate **IV** erfolgen, die sich mittels konventioneller Methoden in die Enantiomeren **IVa** und **IVb** spalten lassen (siehe z.B. *Tetrahedron Asymmetry* **1999,** *10*, 1079).
Tabelle 1 gibt einen Überblick über repräsentative Beispiele für Verbindungen der Formel **I,** die nach den genannten Methoden erzeugt und spektroskopisch charakterisiert wurden. Eine detaillierte Beschreibung für die Herstellung der Diastereomerenmischung (*R*_{*a*},*R*_{*C*}*)-Quinaphos und der reinen Diastereomeren (*R*_{*a*},*R*_{*C*})-Quinaphos und (*R*_{*a*},*S*_{*C*})-Quinaphos (Quinaphos: R¹ = R² = Ph, R³ = *n*-Bu, R⁴-R⁵ = 1,1'-Binaphth-2,2'-diyl) ist in Beispiel 1 zu finden. Die Zuordnung der absoluten Konfiguration des chiralen Zentrums in 2-Position des Chinolingerüsts beruht auf dem Vergleich NMR-spektroskopischer Daten mit verwandten chiralen Derivaten des Chinolins (*Eur. J. Inorg. Chem.* **1999,** *8,* 1203) und ist mit der entsprechenden Unsicherheit behaftet.

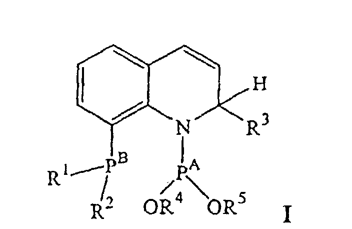

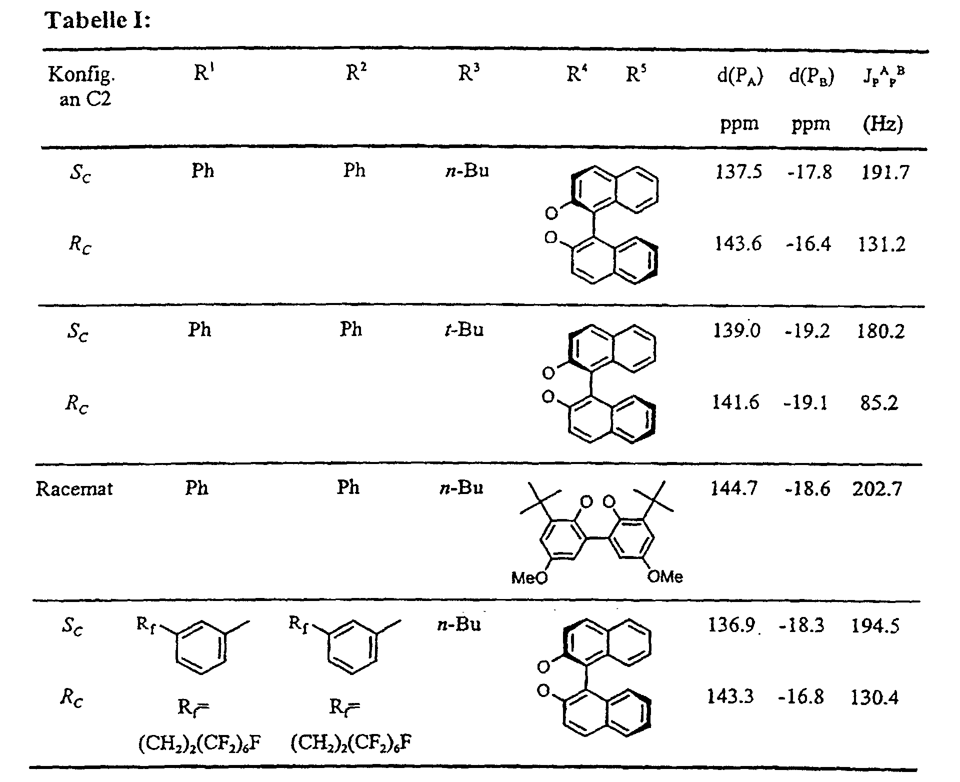

### Anwendung in der Katalyse:

Die chiralen Phosphorverbindungen **I** können in enantiomerenreiner Form, als Diastereomerengemisch oder in Form der reinen Diastereomeren als effektive Katalysatoren oder Katalysatorbestandteile in der Synthese optisch aktiver Produkte verwendet werden. Besonders bevorzugt sind Synthesen, bei denen die Verbindungen **I** als Bestandteile ("Liganden") von Übergangsmetallkatalysatoren eingesetzt werden. Solche Katalysatoren enthalten ein oder mehrere Übergangsmetallzentren, die verschieden oder identisch sein können. Bevorzugte Übergangsmetallzentren, die verschieden oder identisch sein können. Bevorzugte Metalle sind Cu, Ag, Au, Ni, Pd, Pt, Co, Rh, Ir, Fe, Ru, Os, Mn, Re, Cr, Mo, W, Ti, oder Zr. Besonders bevorzugt sind Cu, Ni, Pd, Pt, Rh, Ir oder Ru.
Die Katalysatoren können in Form isolierter Verbindungen, die bereits das Metall und den Liganden **I** enthalten, eingesetzt werden, oder *in situ* aus **I** und geeigneten metallhaltigen Komponenten gebildet werden. Als metallhaltige Komponenten können die Metalle selbst, einfache Salze, oder Komplexverbindungen der entsprechenden Metalle verwendet werden. Das molare Verhältnis zwischen Ligand **I** und Metallzentrum kann für die jeweilige Reaktion optimal angepaßt werden und liegt in der Regel zwischen 1:1 und 10:1.
Die katalytischen Synthesen unter Verwendung der Liganden **I** können entweder in Abwesenheit oder Anwesenheit eines Lösungsmittels durchgeführt werden, wobei das Lösungsmittel einen positiven Einfluß auf die Aktivität oder die Enantioselektivität haben kann, oder die Trennung von Produkt und Katalysator erleichtern kann. Als Lösungsmittel können typische organische Lösungsmittel wie z.B. Benzol, Toluol, Methylenchlorid, Ethanol, Tetrahydrofuran, oder Diethylether verwendet werden. Auch Wasser ist als Lösungsmittel geeignet, wenn der Ligand durch geeignete polare Substituenten (z.B. -COOH, NH₃⁺, SO₃⁻, siehe *Angew. Chem.* **1993,** *105*, 1588) eine ausreichende Löslichkeit in Wasser aufweist. Die Reaktionen können auch in überkritischem Kohlendioxid als Lösungsmittel durchgeführt werden, wenn durch geeignete Substituenten (z.B. Perfluoralkylreste, siehe PCT-Anmeldung WO 98/32533) eine ausreichende Löslichkeit sichergestellt wird. Die Liganden **I** können zur leichteren Abtrennung von den Reaktionsprodukten mittels bekannter Methoden (Adsorption, Einschluß, kovalente Verknüpfung: *Synthesis* **1997,** 1217.) an feste Träger gebunden werden. Die Anwendungsbreite der Liganden **I** beinhaltet asymmetrische Reduktionen (z.B. Hydrierung, Transfer-Hydrierung), asymmetrische C-C-Verknüpfung (z.B. Hydroformylierung, Heck-Kupplung, Allylische Alkylierung, Hydrocyanierung, Hydrovinylierung, Polymerisation) und asymmetrische Knüpfung von Bindungen zwischen Kohlenstoff und Heteroatomen (z. B. Hydroborierung, Hydrosilylierung, Hydroaminierung, Hydrophosphinierung), wie in den folgenden Beispielen anhand des Quinaphos-Liganden verdeutlicht wird.

### Enantioselektive Hydroformylierung mit Liganden I:

Die enantioselektive Hydroformylierung ist eine effiziente Methode zur Synthese chiraler, nicht-racemischer Aldehyde aus Olefinen (*Catalytic Asymmetric Synthesis,* Hrsg.: I. Ojima, VCH, Weinheim, 1993, S. 273ff). Dieser Reaktionstyp hat insbesondere als möglicher Zugang zu chiralen Bausteinen für die Produktion von Aromastoffen, Kosmetika, Pflanzenschutzmitteln, Lebensmittelzusatzstoffen (Vitamine) und Pharmazeutika großes Interesse gefunden (*Chirality* **1991,** 3, 355). Besonders erwähnt sei hier die Darstellung der entzündungshemmenden und schmerzstillenden Wirkstoffe Ibuprofen und Naproxen durch Oxidation der entsprechenden Aldehyde, welche mit Hilfe der enantioselektiven Hydroformylierung aus Vinylarenen gewonnen werden können. Neben der Enantioselektivität ist in dieser Reaktion auch die Chemoselektivität (Nebenreaktion v.a. Hydrierung) und die Regioselektivität zugunsten des verzweigten chiralen Aldehyds von besonderer Bedeutung. Im Falle des Quinaphos werden die besten Enantioselektivitäten in der Hydroformylierung von Styrol mit dem (*R*_{*a*},*R*_{*C*})-Diastereomer gebildet (Beispiel 2-4). Die Hydrierung spielt als unerwünschte Nebenreaktion keine Rolle. Im Vergleich zu Liganden mit vergleichbarer Aktivität und Enantioselektivität werden die höchsten Regioselektivitäten zugunsten des chiralen Aldehyds erreicht (*Chem. Rev.* **1995**, *95*, 2485-2506)
Bevorzugte Katalysatoren für die Hydroformylierung werden auf Basis der Metalle Fe, Co, Ir, Ru, Pt, Rh, besonders bevorzugt auf Basis von Pt und Rh gebildet. Das molare Verhältnis Ligand/Metall sollte zwischen 1:1 und 10:1, bevorzugt zwischen 1:1 und 4:1 liegen.
Das molare Verhältnis von Substrat und Katalysator kann in weiten Grenzen variiert werden, bevorzugt wird ein Verhältnis zwischen 100:1 bis 10 000:1 verwendet. Die Gase H₂ und CO können entweder getrennt oder als Gemisch dem Reaktor zugefügt werden. Der Partialdruck der einzelnen Gase liegt im Bereich von 1-100 bar. Der Gesamtdruck an Synthesegas kann im Bereich von 1-200 bar, bevorzugt im Bereich von 10-100 bar liegen. Die Reaktionstemperatur kann in weiten Bereichen variiert werden und liegt zwischen -20°C und 150°C, bevorzugt zwischen 20°C und 80°C.

### Enantioselektive Hydrierung mit Liganden I:

Die enantioselektive Hydrierung ist eine effiziente Methode zur Synthese chiraler, nicht-racemischer organischer Verbindungen (*Catalytic Asymmetric Synthesis,* Hrsg.: I. Ojima, VCH, Weinheim, 1993, S. 1ff), die vor allem zur Herstellung von biologisch aktiven Wirkstoffen große Bedeutung hat. Die enantioselektive Hydrierung ist für eine Vielzahl funktioneller Gruppen bekannt, insbesondere für Substrate mit prochiralen C=C, C=N oder C=O Doppelbindungen. Die Hydrierung von Dehydroaminosäuren ist ein attraktiver Zugang zu natürlichen und nicht-natürlichen Aminosäuren und hat beispielsweise in der Herstellung von L-Dopa, einem Medikament gegen die Parkinson'sche Krankheit, bereits technische Anwendung gefunden (*Topics in Catalysis* **1998,** *5*, 3).
Bevorzugte Katalysatoren für die Hydrierung mit Liganden **I** werden auf Basis der Metalle Pd, Pt, Co, Ir, Rh und Ru gebildet. Das molare Verhältnis Ligand/Metall sollte zwischen 1:1 und 10:1, bevorzugt zwischen 1:1 und 2.5:1 liegen. Im Falle des Quinaphos werden die besten Enantioselektivitäten in der Hydrierung von Itaconsäuredimethylester mit dem (*R*_{*a*},*S*_{*C*})-Diastereomer erreicht (Beispiele 5,7).
Das molare Verhältnis von Substrat und Katalysator kann in weiten Grenzen variiert werden und liegt bevorzugt zwischen 100:1 und 100 000:1. Das Katalysatorsystem Rh(I)/Quinaphos zeigt eine für Rhodiumkatalysatoren bemerkenswert hohe Aktivität und Lebensdauer (Beispiel 11). Die Hydriergeschwindigkeit von mindestens 36 000 katalytischen Zyklen pro Stunde ist erheblich höher als die typischer Weise für Katalysatoren auf Basis von Rhodiumkatalysatoren mit Phosphorverbindungen beobachteten Aktivitäten (ca. 200 Zyklen pro Stunde, *J. Chem. Soc. (A)* **1967,** 1574.)
Der Partialdruck des Wasserstoffs während der Hydrierung sollte im Bereich von 0.3-200 bar, bevorzugt zwischen 10-100 bar liegen. Die Reaktionstemperatur kann in weiten Bereichen variiert werden und liegt zwischen -20°C und 150°C, bevorzugt zwischen 20°C und 60°C.

### Enantioselektive Hydroborierung mit Liganden I:

Die enantioselektive Hydroborierung ist ein typisches Beispiel für eine Reaktion unter Ausbildung einer Kohlenstoff-Heteroatom Bindung. Sie hat reges Interesse gefunden, da die entstehenden Borane interessante Zwischenstufen für weitere Synthesen (z.B. Bildung von chiralen Alkoholen, C-C-Verknüpfung etc.) sind (*Tetrahedron* **1997,** 53, 4957). Neben der Enantioselektivität der C-B-Verknüpfung sind für diese Reaktion auch die Chemoselektivität (Nebenreaktion v.a. Reduktion) und die Regioselektivität wichtige Kenngrößen.
Bevorzugte Katalysatoren für die Hydroborierung mit Liganden **I** werden auf Basis von Rh gebildet. Das molare Verhältnis Ligand/Metall sollte zwischen 1:1 und 4:1, bevorzugt zwischen 1:1 und 2:1 liegen (Beispiel 14,17).
Das molare Verhältnis von Substrat und Katalysator kann in weiten Grenzen variiert werden und liegt bevorzugt zwischen 100:1 und 10 000:1. Die Reaktionstemperatur kann in weiten Bereichen variiert werden und liegt zwischen -80°C und 100°C, bevorzugt zwischen 20°C und 80°C.

### Beispiele

### Beispiel 1: Synthese von (Rₐ,R_{C}*)-Quinaphos, (Rₐ,R_{C})-Quinaphos und (Rₐ,S_{C})-Quinaphos

In einem Schlenkgefäß wurde zu 8-Diphenylphosphinochinolin (1.0 g, 3.2 mmol) in THF (40 mL) bei -78°C eine Lösung von n-BuLi in Pentan (1.6 M, 2 mL) mit einer Spritze zugegeben. Die Lösung wurde auf 0°C erwärmt und 30 min. bei dieser Temperatur gerührt. Die erhaltene dunkelrote Lösung wurde in einen gekühlten Tropftrichter überführt und bei -30°C langsam zu einer Lösung von (R)-1,1'-Binaphthyl-2,2'-dioxy)-chlorophosphin in THF (20 mL) getropft. Die Reaktionsmischung wurde unter Rühren über Nacht auf Raumtemperatur erwärmt. Nach Entfernen des Lösungsmittels im Vakuum wurde der Rückstand mit Toluol (50 mL) extrahiert. Das Extraktionsmittel wurde im Vakuum entfernt und der Rückstand aus Methylenchlorid/Ethanol bei -20°C umkristallisiert. Es wurden 1.24 g (1.8 mmol, 56% d. Th.), (*R*_{*a*},*R*_{C}*)-Quinaphos als 1:1 Diastereomerenmischung in Form eines weißen mikrokristallinen Pulvers erhalten.
Zur Trennung der Diastereomeren wurden 0.24 g (*R*ₐ,*R*_{C}*)-Quinaphos an ausgeheiztem Kieselgel (Merck, Typ 9385, 230-400 mesh) chromatographiert. Mit Methylenchlorid/Pentan (1:5) wurde als erste Fraktion (*R*ₐ,*S*_{C})-Quinaphos (0.12 g, 100% d. Th.) eluiert. Anschließend wurde mit reinem Methylenchlorid (*R*ₐ,*R*_{C})-Quinaphos (0.05g, 42% d. Th.) eluiert.
Ausgewählte analytische Daten:
(*R*ₐ,*S*_{C})-Quinaphos:
¹H NMR (C₆D₆): 8 = 7.86 (d, *J=* 8.7 Hz, 1H, Ar-H), 7.70-7.39 (m, 11H, Ar-H), 7.31 (m, 1H, Ar-H), 7.10-6.84 (m, 11H, Ar-H), 7.78 (m, 1H, Ar-H), 6.39 (d, ³*J*= 9.5 Hz, 1H, CH=CH), 5.72 (dd, ³*J*= 9.5 Hz, ³*J* = 5.6 Hz, 1H, CH=CH), 4.01 (m, 1H, CH), 1.40-0.90 (m, 6H, CH₂), 0.61 (t, ³*J*= 7.2 Hz, 3H, CH₃).
³¹P-NMR (C₆D₆): siehe Tabelle 1;
(*R*_{*a*},*R*_{*C*})-Quinaphos:
¹H-NMR (C₆D₆): δ = 7.63-7.57 (m, 6H, Ar-H), 7.51-7.43(m, 2H, Ar-H), 7.28-7.22 (m, 4H, Ar-H), 7.126.94 (m, 12H, Ar-H), 7.79 (m, 1H, Ar-H), 6.18 (d, ³*J* = 9.6 Hz, 1H, CH=CH), 5.56 (dd, ³*J* = 9.6 Hz, ³*J* = 5.7 Hz, 1H, CH=CH), 3.88 (m, 1H, CH), 1.40-0.88 (m, 6H, CH₂), 0.73 (t, ³*J*= 7.2 Hz, 3H, CH₃).
³¹P-NMR (C₆D₆): siehe Tabelle 1;
Die Diastereomerenmischung (*R*_{*a*},*R*_{*C*}*)-Quinaphos zeigt die beiden Datensätze der einzelnen Diastereomeren im Verhältnis 1:1.

### Beipiel 2-4: Enantioselektive Hydroformylierung von Styrol

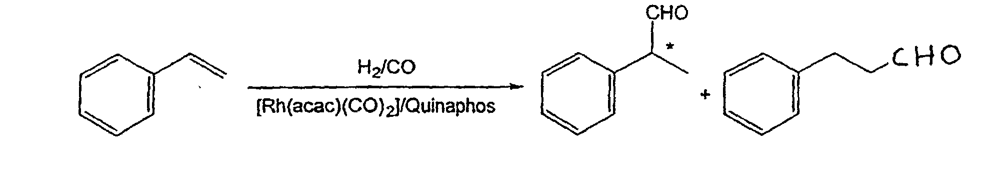

In einem mit Sichtfenstern, einem Manometer, Ventilen, und je einem Thermofühler für Mantel- und Innentemperatur ausgestatten Stahlautoklaven (*V*=11.4 mL) wurde der Komplex [Rh(acac)(CO)₂] (0.52 mg, 2 x 10⁻³ mmol, acac = Acetylacetonat) und Quinaphos (5.5 mg, 8 x 10⁻³ mmol) vorgelegt. Anschließend wurde Styrol (0.5 mL) zugegeben (molares Verhältnis Substrat/Rhodium = S/Rh). Synthesegas (CO/H₂=1:1) wurde mit dem Druck von 100 bar bei Raumtemperatur aufgepreßt und auf 40 °C erhitzt. Nach der Reaktionszeit t wurde der Reaktor auf Raumtemperatur abgekühlt, entspannt und die Reaktionsmischung nach konventionellen Methoden aufgearbeitet. Umsatz, Chemoselektivität, Regioselektivität zugunsten des verzweigten Aldehyds und Enantiomerenüberschuß (ee) wurden mittels Gaschromatographie (HP 5890 mit FID, Säule: Ivadex 7, Injektortemp.: 240°C, Säulentemp.: 60-200°C; Detektortemp.: 300°C, Carriergas: H₂) bestimmt.

| Beispiel | S/Rh | Quinaphos | L/Rh | *t* (h) | Umsatz (%) | Chemosel.(%) | Regiosel.(%) | ee (%) |
|---|---|---|---|---|---|---|---|---|
| 2 | 2200 | (*R*_{*a*}*,R*_{*C*}*) | 4 | 90 | 54.8 | >99 | 96.3 | 35.6 (*S*) |
| 3 | 2200 | (*R*_{*a*}*,S*_{*C*}) | 4 | 74 | 79.3 | >99 | 96.0 | 4.8 (*S*) |
| 4 | 2200 | (*R*_{*a*}*,R*_{*C*}) | 4 | 70 | 75 | >99 | 96.7 | 74.0 (S) |

### Beispiel 5-10: Enantioselektive Hydrierung von Itaconsäuredimethylester

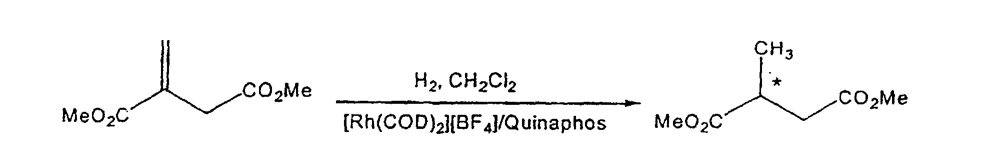

In einem mit Sichtfenstern, einem Manometer, Ventilen und je einem Thermofühler für Mantel- und Innentemperatur ausgestatteten Stahlautoklaven (*V*=11.4 mL) wurde der Komplex [Rh(COD)₂][BF₄] (2 x 10⁻³ mmol Rh, COD = 1,5-Cyclooctadien) und soviel Quinaphos in Methylenchlorid (2-6 mL) gelöst, daß das gewünschte Quinaphos-zu-Rhodium-Verhältnis (L/Rh) erhalten wurde. Anschließend wurde die entsprechende Menge Substrat (ca. 0.32-1.90 g) zugegeben (molares Verhältnis Substrat/Rhodium = S/Rh siehe Tabelle). Wasserstoff wurde mit dem Druck *p*H₂ bei Raumtemperatur (RT) aufgepreßt. Nachdem die Lösung bei der angegebenen Reaktionstemperatur *T* während der Reaktionszeit *t* kräftig gerührt wurde, wurde entspannt und nach konventionellen Methoden aufgearbeitet. Umsatz und Enantiomerenüberschuß (ee) wurden mittels Gaschromatographie (HP 5890 mit FID, Säule: γ-Cyclodextrin, Injektortemp.: 180°C, Säulentemp.: 60-87°C; Detektortemp.: 250°C, Carriergas: H₂) bestimmt.

| Beispiel | S/Rh | Quinaphos | L/Rh | *T* (°C) | *p*_{H2} (bar) | *t* (h) | Umsatz (%) | ee -(%) |
|---|---|---|---|---|---|---|---|---|
| 5 | 1000 | ***(R,R)*** | 1.1 | RT | 30 | 24 | >99 | **64.2** (*R*) |
| 6 | 1000 | ***(R,R)*** | 2.2 | RT | 30 | 24 | >99 | **78.8** (*R*) |
| 7 | 1000 | **(*R,S*)** | 1.1 | RT | 30 | 24 | >99 | **95.6** (*R*) |
| 8 | 1000 | **(*R,S*)** | 2.2 | RT | 30 | 24 | >99 | **98.8** (*R*) |
| 9 | 1000 | **(*R,S*)** | 2.2 | 0 | 10 | 144 | 41.5 | **55.0** (*R*) |
| 10 | 1000 | **(*R,S*)** | 2.2 | RT | 0.8 | 24 | 5.5 | **24.6** (*R*) |

### Beispiel 11: Katalysatoraktivität und Lebensdauer.

Der Versuch wurde entsprechend Beispiel 5-10 durchgeführt, wobei jedoch der isolierte Komplex [{(*R,S*)-Quinaphos)}Rh(COD)][BF₄] als Katalysator eingesetzt wurde. Die Reaktion wurde bereits nach 5 min. durch Entspannen abgebrochen, eine Probe entnommen und analysiert (11a). Anschließend wurde erneut Substrat zu der Reaktionslösung gegeben und H₂ aufgepreßt. Nach 10 min. wurde wiederum entspannt und analysiert (11b). In beiden Fällen erfolgte vollständige Hydrierung, so daß als Untergrenze der Aktivität eine Katalysatoraktivität (TOF = katalytische Zyklen pro Stunde) von 36 000 h⁻¹ abgeschätzt werden kann.

| Beispiel | S/Rh | *T* (°C) | *p*_{H₂} (bar) | *t* (min) | Conv. (%) | TOF (h⁻¹) | ee (%) |
|---|---|---|---|---|---|---|---|
| 11a | 1000 | 27 | 50 | **5** | >99 | **>12000** | **98.2** (*R*) |
| 11b | 6000 | 27 | 70 | **10** | >99 | **>36000** | **99.4** (*R*) |

### Beispiel 12-13: Hydrierung von N-Acetamidoacrylsäurmethylester.

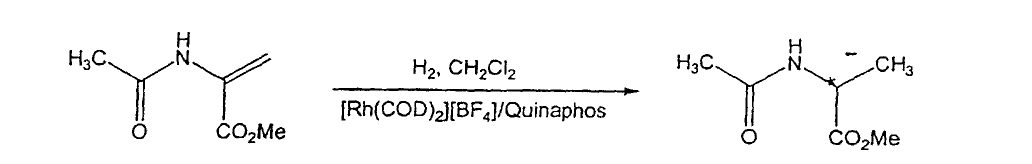

Die Beispiele 12-13 wurden analog zu den Beispielen 5-10 durchgeführt.

| Beispiel | Quinaphos | S/Rh | L/Rh | *T*/°C | *p*_{H₂} (bar) | *t*/h | Conv. (%) | ee (%) |
|---|---|---|---|---|---|---|---|---|
| 12 | **(*R,S*)** | 1000 | 2.2^{a} | RT | 30 | 85 | 8.0 | **12.4** (*S*) |
| 13 | **(*R,S*)** | 1000 | 1.0^{b} | RT | 30 | 25 | >99 | **97.8** (*S*) |

| | | | | | | | | |
|---|---|---|---|---|---|---|---|---|
| *a) in situ* Katalysator aus Quinaphos und [Rh(COD)₂][BF₄]; | | | | | | | | |
| b) isolierter Komplex [(Quinaphos)Rh(COD)][BF₄] als Katalysator. | | | | | | | | |

### Beispiel 14: Enantioselektive Hydroborierung von Styrol

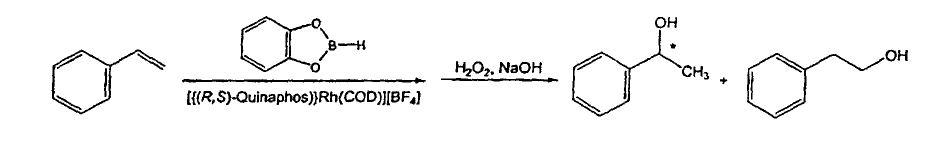

In einem Schlenkgefäß wurde [{(*R,S*)-Quinaphos}Rh(COD)][BF₄] (4 x 10⁻³ mmol) und soviel Quinpahos vorgelegt, daß das gewünschte Quinaphos-zu-Rhodium-Verhältnis (L/Rh) erhalten wurde. Der Katalysator wurde in 3 mL Lösungsmittel (LM) gelöst und Styrol (0.045 mL) zugegeben (molares Verhältnis Substrat/Rhodium = S/Rh siehe Tabelle). Nach Zugabe von Catecholboran (0.048 mL) wurde die Lösung bei der angegebenen Reaktionstemperatur *T* während der Reaktionszeit *t* gerührt und anschließend nach konventionellen Methoden oxidativ aufgearbeitet. Umsatz, Chemo- und Regioselektivität sowie Enantiomerenüberschuß (ee) des sekundären Alkohols wurden mittels Gaschromatographie (HP 5890 mit FID, Säule: Ivadex 7, Injektortemp.: 220°C, Säulentemp.: 60-160°C; Detektortemp.: 250°C, Carriergas: H₂) bestimmt.

| Beispiel | S/Rh | L/Rh | LM | *T* (°C) | *t* (h) | Umsatz (%) | Chemosel (%) | Regiosel. (%) | ee (%) |
|---|---|---|---|---|---|---|---|---|---|
| 14 | 100 | 1 | Toluol | 25 | 3 | >99 | >99 | 91.8 | 30.4 (*S*) |
| 15 | 100 | 1 | Toluol | -20 | 14 | 48.5 | 93.7 | 76.3 | 19.0 (*S*) |
| 16 | 100 | 1 | Toluol | -78 | 20 | 4.7 | 56.4 | 88.9 | 15.3 (*S*) |
| 17 | 100 | 2 | Toluol | 25 | 3 | 3.8 | 93.5 | 78.4 | 20.8 (*S*) |
| 18 | 100 | 2 | THF | 25 | 3 | 2.4 | 92.5 | 69.1 | 5.0 (*S*) |

## Patentansprüche

1. Chirale Phosphorverbindungen, der allgemeine Formel **I,** 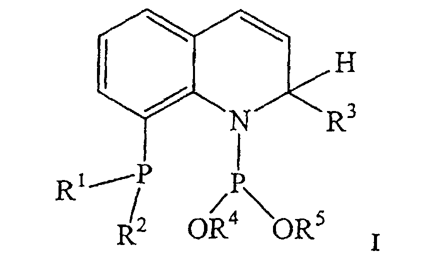 wobei R¹, R², R³, R⁴, R⁵ chirale oder achirale organische Reste sind, die sich von substituierten oder unsusbstituierten geradkettigen, verzweigten, oder cyclischen aliphatischen oder aromatischen Gruppen ableiten, und die im Falle der Paare R¹/R² und R⁴/R⁵ miteinander verbunden sein können.

2. Chirale Phosphorverbindungen nach Anspruch 1, wobei die Reste R¹, R², R³, R⁴, oder R⁵ unabhängig voneinander gewählt werden können aus den Gruppen Methyl, Ethyl, *n*-Propyl, *n*-Butyl, Hexyl, F(CF₂)ₘ(CH₂)ₙ- (m = 1-10, n = 0-4), Cyclohexyl, Menthyl, Allyl, Benzyl, -CH₂O(CH₂)₂OCH₃, Phenyl, Tolyl, Anisyl, Trifluormethylphenyl, F(CF₂)ₘ(CH₂)ₙC₆H₄- (m = 1-10, n = 0-4), Bis-(trifluormethyl)-phenyl, Chlorphenyl, Pentafluorphenyl, Hydroxyphenyl, Carboxyphenyl, NaO₃SC₆H₄-, Naphthyl, Fluorenyl, Pyridyl oder Furyl.

3. Chirale Phosphorverbindungen nach Anspruch 1-2, wobei die Reste R¹ und R² so miteinander verbunden sind, daß substituierte oder unsubstituierte, chirale oder achirale Brücken entstehen, die sich von den Gerüsten -(CH₂)ₙ-(n=2-4), -CH(CH₃)CH(CH₃)-, -CH(CH₃)CH₂CH(CH₃)-, 1,1'-Biphen-2,2'-diyl oder 1,1'-Binaphth-2,2'-diyl ableiten.

4. Chirale Phosphorverbindungen nach Anspruch 1-3, wobei die Reste R⁴ und R⁵ unter Verwendung der Alkohole Methanol, Ethanol, *iso*-Propanol, Benzylalkohol, Cyclohexanol, Allylalkohol, Phenol, Methylphenol, Chlorphenol, Naphthol, Furfurol, Ethylenglycol, 1,3-Propandiol, 1,3-Pentandiol, Cyclohexandiol, Gylcerin, Monosaccharide, Oligosaccharide, Catechol, 2,2'-Dihydroxy-1,1 '-Biphenyl, 3,3',5,5'-Tetra-*tert*.-Butyl-2,2'-Dihydroxy-1,1'-Biphenyl, 3,3'-Di-*tert*.-Butyl-2,2'-Dihydroxy-5,5'-Dimethoxy-1,1'-Biphenyl, 5,5'-Dichloro-4,4',6,6'-Tetramethyl-2,2'-Dihydroxy-1,1'-Biphenyl, oder 2,2'-Dihydroxy-1,1'-Binaphthyl eingeführt werden.

5. Verwendung der unter Anspruch 1-4 genannten Phosphorverbindungen zur Herstellung optisch aktiver Produkte, wobei die Phosphorverbindungen als Katalysatoren oder Katalysatorbestandteile eingesetzt werden.

6. Verwendung nach Anspruch 5, wobei die Katalysatoren aus Phosphorverbindungen der Formel **I** und einem Übergangsmetall oder einer Übergangsmetallverbindung bestehen.

7. Verwendung nach Ansprüchen 5-6, wobei die optischen Produkte durch enantioselektive Hydroformylierung gebildet werden.

8. Verwendung nach Ansprüchen 5-6, wobei die optisch aktiven Produkte durch enantioselektive Hydrierung gebildet werden.

9. Verwendung nach Ansprüchen 5-6, wobei die optisch aktiven Produkte durch enantioselektive Hydroborierung gebildet werden.

## Claims

1. Chiral phosphorus compounds of general formula I 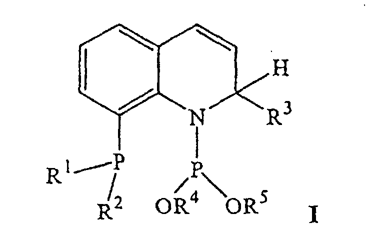 wherein R¹, R², R³, R⁴, R⁵ are chiral or achiral organic residues which are derived from substituted or unsubstituted straight or branched chain or cyclic aliphatic or aromatic groups and which, in the case of the pairs R¹/R² and R⁴/R⁵, may be interconnected.

2. The chiral phosphorus compounds according to claim 1, wherein residues R¹, R², R³, R⁴ or R⁵ can be independently selected from the groups methyl, ethyl, n-propyl, n-butyl, hexyl, F(CF₂)ₘ(CH₂)ₙ- (m = 1-10, n = 0-4), cyclohexyl, menthyl, allyl, benzyl, -CH₂O(CH₂)₂OCH₃, phenyl, tolyl, anisyl, trifluoromethylphenyl, F(CF₂)ₘ(CH₂)ₙC₆H₄- (m = 1-10, n = 0-4), bis(trifluoromethyl)phenyl, chlorophenyl, pentafluorophenyl, hydroxyphenyl, carboxyphenyl, NaO₃SC₆H₄-, naphthyl, fluorenyl, pyridyl or furyl.

3. The chiral phosphorus compounds according to claim 1 or 2, wherein residues R¹ and R² are interconnected to form substituted or unsubstituted chiral or achiral bridges which are derived from the skeletons -(CH₂)ₙ- (n = 2-4), -CH(CH₃)CH(CH₃)-, -CH(CH₃)CH₂CH(CH₃)-, 1,1'-bipheny-2,2'-diyl or 1,1'-binaphth-2,2'-diyl.

4. The chiral phosphorus compounds according to claims 1 to 3, wherein residues R⁴ and R⁵ are introduced using the alcohols methanol, ethanol, isopropanol, benzyl alcohol, cyclohexanol, allyl alcohol, phenol, methylphenol, chlorophenol, naphthol, furfurol, ethylene giycol, 1,3-propanediol, 1,3-pentanediol, cyclohexanediol, glycerol, monosaccharides, oligosaccharides, catechol, 2,2'-dihydroxy-1,1'-biphenyl, 3,3',5,5'-tetra-tert-butyl-2,2'-dihydroxy-1,1'-biphenyl, 3,3'-di-tert-butyl-2,2'-dihydroxy-5,5'-dimethoxy-1,1'-biphenyl, 5,5'-dichloro-4,4',6,6'-tetramethyl-2,2'-dihydroxy-1,1'-biphenyl or 2,2'-dihydroxy-1,1'-binaphthyl.

5. Use of the phosphorus compounds as defined in claims 1 to 4 for the preparation of optically active products, wherein said phosphorus compounds are employed as catalysts or catalyst components.

6. The use according to claim 5, wherein said catalysts consists of phosphorus compounds of formula I and a transition metal or a transition metal compound.

7. The use according to claim 5 or 6, wherein said optically active products are formed by enantioselective hydroformylation.

8. The use according to claim 5 or 6, wherein said optically active products are formed by enantioselective hydrogenation.

9. The use according to claim 5 or 6, wherein said optically active products are formed by enantioselective hydroboration.

## Revendications

1. Composés de phosphore chiraux de formule générale I : 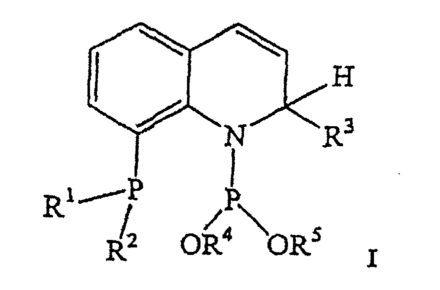 dans laquelle R¹, R², R³, R⁴, R⁵ sont des radicaux organiques chiraux ou achiraux, qui dérivent de groupes aliphatiques ou aromatiques substitués ou non substitués, à chaîne droite, ramifiée ou cyclique et qui peuvent être reliés l'un à l'autre dans le cas de la paire R¹/R² et de la paire R⁴/R⁵.

2. Composés de phosphore chiraux selon la revendication 1, dans lesquels les radicaux R¹, R², R³, R⁴ ou R⁵ peuvent être choisis indépendamment l'un de l'autre parmi les groupes méthyle, éthyle, *n*-propyle, *n*-butyle, hexyle, F(CF₂)ₘ(CH₂)ₙ- (m=1-10, n=0-4), cyclohexyle, menthyle, allyle, benzyle, -CH₂O(CH₂)₂OCH₃, phényle, tolyle, anisyle, trifluorométhylphényle, F(CF₂)ₘ(CH₂)ₙC₆H₄- (m=1-10, n=0-4), bis-(trifluorométhyl)-phényle, chlorophényle, pentafluorophényle, hydroxyphényle, carboxyphényle, NaO₃SC₆H₄-, naphtyle, fluorényle, pyridyle ou furyle.

3. Composés de phosphore chiraux selon les revendications 1 et 2, dans lesquels les radicaux R¹ et R² sont reliés l'un à l'autre de sorte qu'ils forment des ponts chiraux ou achiraux substitués ou non substitués, qui dérivent des squelettes -(CH₂)ₙ- (n=2-4), -CH(CH₃)CH(CH₃)-, -CH(CH₃)CH₂CH(CH₃)-, 1,1'-biphén-2,2'-diyle ou 1,1'-binapht-2,2'-diyle.

4. Composés de phosphore chiraux selon les revendications 1 à 3, dans lesquels les radicaux R⁴ et R⁵ sont introduits en utilisant les alcools suivants : le méthanol, l'éthanol, l'isopropanol, l'alcool benzylique, le cyclohexanol, l'alcool allylique, le phénol, le méthylphénol, le chlorophénol, le naphtol, le furfurol, l'éthylèneglycol, le 1,3-propanediol, le 1,3-pentanediol, le cyclohexanediol, la glycérine, les monosaccharides, les oligosaccharides, le catéchol, le 2,2'-dihydroxy-1,1'-biphényle, le 3,3',5,5'-tétra*-tert*-butyl-2,2'-dihydroxy-1,1'-biphényle, le 3,3'-di*-tert*-butyl-2,2'-dihydroxy-5,5'-diméthoxy-1,1'-biphényle, le 5,5'-dichloro-4,4',6,6'-tétraméthyl-2,2'-dihydroxy-1,1'-biphényle ou le 2,2'-dihydroxy-1,1'-binaphtyle.

5. Utilisation des composés de phosphore mentionnés dans les revendications 1 à 4 pour la fabrication de produits optiquement actifs, dans laquelle les composés de phosphore sont mis en oeuvre comme catalyseurs ou composants de catalyseurs.

6. Utilisation selon la revendication 5, dans laquelle les catalyseurs sont constitués de composés de phosphore de formule I et d'un métal de transition ou d'un composé de métal de transition.

7. Utilisation selon les revendications 5 et 6, dans laquelle les produits optiques sont formés par hydroformylation énantiosélective.

8. Utilisation selon les revendications 5 et 6, dans laquelle les produits optiquement actifs sont formés par hydruration énantiosélective.

9. Utilisation selon les revendications 5 et 6, dans laquelle les produits optiquement actifs sont formés par hydroboration énantiosélective.
